(19) Europäisches Patentamt
European Patent Office
Office européen des brevets

(11) **EP 0 919 618 A1**

(12) **EUROPÄISCHE PATENTANMELDUNG**

(43) Veröffentlichungstag:
**02.06.1999 Patentblatt 1999/22**

(21) Anmeldenummer: 97120891.3

(22) Anmeldetag: **28.11.1997**

(51) Int. Cl.$^6$: **C12N 15/12**, C12N 15/86,
C07K 14/47, C12N 5/10,
A61K 38/17

(84) Benannte Vertragsstaaten:
**AT BE CH DE DK ES FI FR GB GR IE IT LI LU MC
NL PT SE**
Benannte Erstreckungsstaaten:
**AL LT LV MK RO SI**

(71) Anmelder: **Roche Diagnostics GmbH
68305 Mannheim (DE)**

(72) Erfinder:
**Die Erfindernennung liegt noch nicht vor**

(54) **Aktive Hedgehog-Protein-Mutante, Verfahren zur Herstellung und Verwendung**

(57) Ein posttranslational prozessierte Hedgehog-Protein-Mutante, welche

— unter alkylierenden Bedingungen ein Molekulargewicht von 22 ± 1 kDa zeigt,
— unter reduzierenden Bedingungen ein Molekulargewicht von 24 ± 1 kD zeigt,
— in ihrer Aktivität durch Suramin stabilisiert wird,
— bei einer N-terminalen Abspaltung von 8 oder mehr Aminosäuren inaktiviert wird,

— bei einer Inkubation mit 10 mmol/l DTE über 2,5 Stunden bei 37°C zu 90 % oder mehr inaktiviert wird,
— in einer Konzentration von 5 nmol/l, in Gegenwart von Suramin, eine Aktivität für alkalische Phosphatase von ca. 90 nmol pNP/min/mg induziert und
— nicht cholesterinmodifziert ist,

zeigt eine vielfach erhöhte Aktivität.

Abb. 1

**Beschreibung**

Gegenstand der Erfindung ist eine aktive Form eines Hedgehog-Proteins, Verfahren zu dessen rekombinanter Herstellung und dessen therapeutische Verwendung. Unter Hedgehog (hh)-Proteinen versteht man eine Familie von sekretierten Signalproteinen, die verantwortlich sind für die Ausbildung einer Vielzahl von Strukturen in der Embryogenese (J.C. Smith, Cell 76 (1994) 193 - 196, N. Perrimon, Cell 80 (1995) 517 - 520, C. Chiang et al., Nature 83 (1996) 407, M.J. Bitgood et al., Curr. Biol. 6 (1996) 296, A. Vortkamp et al., Science 273 (1996) 613, C.J. Lai et al., Development 121 (1995) 2349). Bei der Biosynthese wird nach Abspaltung der Signalsequenz und autokatalytischer Spaltung eine 20 kD N-terminale Domäne und eine 25 kD C-terminale Domäne erhalten. Das N-terminale Fragment ist Lipid-modifiziert (J.A. Porter et al., Science 274 (1996) 255 - 259. In höheren Lebewesen besteht die hh-Familie zumindest aus drei Mitgliedern, nämlich Sonic, Indian und Desert hh (Shh, Ihh, Dhh; M. Fietz et al., Development (Suppl.) (1994) 43 - 51). Für Hedgehog-Proteine, welche rekombinant hergestellt wurden, wurde eine unterschiedliche Aktivität nach Herstellung in Prokaryonten und Eukaryonten beobachtet (M. Hynes et al., Neuron 15 (1995) 35 - 44 und T. Nakamura et al., Biochem. Biophys. Res. Comm. 237 (1997) 465 - 469.

Hynes et al. vergleichen die Aktivität von hh im Überstand von transformierten „human embryonic kidney 293 cells" (eukaryontischer hh) mit aus E.coli hergestellten hh und finden eine vierfach höhere Aktivität des hh aus den Überständen der Nierenzellinie. Als Ursache dieser erhöhten Aktivität wird ein potentieller zusätzlicher „acessory factor", der nur in eukaryontischen Zellen exprimiert wird, eine posttranslationale Modifikation, ein unterschiedlicher N-Terminus, der aus E.coli isolierte hh enthält 50 % eines hhs, welcher zwei zusätzliche N-terminale Aminosäuren (Gly-Ser) trägt, oder um 5 - 6 Aminosäuren verkürst ist, oder in einem höheren Aggregatzustand (z. B. durch Bindung an Nikkel-Agarose beads) diskutiert.

Nakamura et al. vergleichen die Aktivität von shh im Überstand von transformierten „chicken embryo fibroblasts" mit einem aus E.coli isolierten shh Fusionsprotein, das noch einen N-terminalen Polyhistidinteil aufweist. Das shh im Überstand der Fibroblasten hat bezüglich der Stimulation von alkalischer Phosphatase (AP) in C3H10T ½ Zellen eine 7fach höhere Aktivität als das gereinigte E.coli Protein. Als Ursache der erhöhten Aktivität werden Moleküle, wie beispielsweise bone morphore genic Proteins (BMPs) diskutiert, die nur im Überstand von eukaryontischen Zellen vorhanden sind und die stärkere Induktion der AP verursacht.

Von Kinto et al., FEBS Letters, 404 (1997) 319 - 323 wurde beschrieben, daß hh sekretierende Fibroblasten bei i.m. Implantation auf Kollagen eine ektopische Knochenbildung induzieren.

Aufgabe der Erfindung ist es, hh-Proteine (Polypeptid) herzustellen, welche eine deutlich verbesserte Aktivität gegenüber den bekannten Formen zeigen.

Die Aufgabe wird gelöst durch eine posttranslational prozessierte Hedgehog-Protein-Mutante (hh-Mutante), welche erhältlich ist durch Expression eines Gens, welches ein Hedgehog-Protein codiert in einem Bacculo-Virus-Expressionssystem, bei einer Fermentation über einen Zeitraum von bis zu ca. 30 Stunden, vorzugsweise von 24 - 27 Stunden, Reinigung des Zellüberstands in Gegenwart eines Proteaseninhibitors und eines nicht-ionischen Detergenz und Isolierung der hh-Mutante, welche an Heparinsepharose und Hydroxylapatit bindet und dadurch gekennzeichnet ist, daß diese hh-Mutante

— unter alkylierenden Bedingungen ein Molekulargewicht von 22 ± 1 kDa zeigt,
— unter reduzierenden Bedingungen ein Molekulargewicht von 24 ± 1 kD zeigt,
— in ihrer Aktivität durch Suramin stabilisiert wird,
— bei einer N-terminalen Abspaltung von 8 oder mehr Aminosäuren inaktiviert wird,
— bei einer Inkubation mit 10 mmol/l 1,4 Dithioerythrit (DTE) über 2,5 Stunden bei 37°C zu 90 % oder mehr inaktiviert wird,
— in einer Konzentration von 5 nmol/l, in Gegenwart von Suramin, eine Aktivität für alkalische Phosphatase von ca. 90 nmol pNP/min/mg induziert und
— nicht cholesterinmodifiziert ist.

Unter Aktivität im Sinne der Erfindung ist die Aktivität an alkalischer Phosphatase zu verstehen, die das Polypeptid in Säugerzellen induzieren kann (Aktivität im alkalischen Phosphatase-test). Dabei wird eine Maus-Fibroblasten-Zellinie in einem Medium, welches fötales Kälberserum enthält, kultiviert. Anschließend wird sterilfiltrierte Probe zugesetzt, nach ca. 5 Tagen die Zellen aufgeschlossen und in Zellysat alkalische Phosphatase über die Spaltung eines chromogenen Substrats (pNP, p-Nitrophenol) bestimmt (J. Asahina, Exp. Cell. Res. 222 (1996) 38 - 47 und T. Nakamura (1997)).

Unter einem Bacculo-Virus-Expressionssystem ist ein Expressionssystem bestehend aus einem Bacculo-Virus-Vektor und einer Insektenzelle als Wirtszelle zu verstehen. Solche Expressionssysteme sind dem Fachmann bekannt und beispielsweise für hh-Proteine von Bumcrot (1995) beschrieben.

Unter einem Hedgehog-Protein, gemäß der Erfindung, ist ein sekretiertes Signalprotein zu verstehen, welches für die Ausbildung einer Vielzahl von Strukturen in der Embryogenese verantwortlich ist. Besonders bevorzugt verwendet wird Sonic, Indian-oder Desert hh (M. Fietz et al. (1994). Bevorzugt wird ein hh-Protein einer Sequenz, wie sie in der EMBL-Datenbank unter Nr. L38518 beschrieben ist, verwendet. Proteine der Hedgehog-Familie zeigen eine ausgeprägte Homologie in der Aminosäuresequenz, weshalb es ebenso bevorzugt ist, solche Nukleinsäuren zu exprimieren, welche für Hedgehog-Proteine codieren, die zu 80 % oder mehr homolog mit der oben genannten Sequenz von Sonic Hedgehog-Protein sind.

Das Sonic Hedgehog-Precursorprotein besteht aus den Aminosäuren 1 - 462 der in der EMBL-Datenbank unter Nr. L38518 beschriebenen Sequenz. Die Aminosäuren 1 - 23 stellen dabei das Signalpeptid dar, die Aminosäuren 24 - 197 die mature Signaldomäne, die Aminosäuren 32 - 197 die um acht Aminosäuren verkürzte Signaldomäne und die Aminosäuren 198 - 462 die autoprozessierte Domäne nach autoproteolytischer Spaltung.

Unter den ersten acht Aminosäuren des Hedgehog-Proteins sind gemäß der Erfindung die ersten acht Aminosäuren des prozessierten Proteins, beispielsweise für Sonic Hedgehog-Protein, die Aminosäuren 24 - 31 zu verstehen.

Überraschenderweise wird bei der rekombinanten Herstellung von Hedgehog-Proteinen im Bacculo-Virus-Expressionssystem eine hochaktive Mutante des Proteins (Aktivität gegenüber shh mindestens um das 10fache, vorzugsweise mindestens um das 100fache erhöht) in der Anfangszeit der Fermentation angereichert. Diese erfindungsgemäße Mutante des Polypeptids kann insbesondere dann in großen Mengen isoliert werden, wenn die Fermentation spätestens nach ca. 30 Std., vorzugsweise nach ca. 24 - 27 Std., abgebrochen wird. Dies ist auch deshalb überraschend, da für die Herstellung von hh-Proteinen im Bacculo-Virus-Expressionssystem bisher eine Fermentationszeit nach Infektion von mindestens 2 Tagen beschrieben wird (Bumcrot et al., Mol. Cell. Biol. (1995) 2294 - 2303). Auch für andere Proteine, die im Baculovirussystem hergestellt werden, wie beispielsweise Rhodopsin Kinase (Cha et al., Proc. Natl. Acad. Sci. USA 94 (1997) 10577 - 10582) ist beschrieben, daß nach 64 - 88 Std. ein Maximum an Protein und Aktivität erzielt wird. Erfindungsgemäß wurde für Hedgehog-Proteine gefunden, daß im Fermentationsüberstand zwar die Menge an Hedgehog-Protein im Zeitraum zwischen 33 und 72 Stunden zwar stark zunimmt, in diesem Zeitraum jedoch nur hh-Protein mit einer Aktivität wie sie aus dem Stand der Technik bekannt ist entsteht. Dagegen ist bei einer reduzierten Fermentationszeit von unter ca. 30 Std. der Anteil an solchem hh-Protein wesentlich (mindestens 5 - 10fach) geringer, wodurch sich die erfindungsgemäße hochaktive hh-Proteinmutante identifizieren und isolieren läßt.

Die erfindungsgemäße hh-Mutante ist sehr sensibel gegen Proteasen, weshalb es bevorzugt ist, den Überstand der Fermentation Proteaseninhibitoren, wie beispielsweise Aprotinin, PMSF oder Pepstatin oder ein Gemisch davon, zuzusetzen.

Weiter ist es bevorzugt, bei der Reinigung nicht-ionische Detergentien, wie beispielsweise Polysorbat (z.B. Triton® X 100), zuzusetzen, da dadurch die erfindungsgemäßen hh-Proteine ebenfalls stabilisiert werden, vorzugsweise, zumindest nach der ersten Grobreinigung, über Heparin-Sepharose.

In einem ersten Schritt zur Reinigung des erfindungsgemäßen Proteins wird zweckmäßig eine Chromatographie an Heparin-Sepharose durchgeführt. Es ist bevorzugt, diese Chromatographie als Stufenelution durchzuführen, d.h. vorzugsweise nach Waschen mit 250 mmol/l NaCl, bei einer Konzentration von mindestens 0,7 mol/l (vorzugsweise 1,2 mol/l) zu eluieren.

Zur Aufreinigung der erfindungsgemäßen hh-Mutante ist es besonders vorteilhaft, eine Hydroxylapatit-Chromatographie durchzuführen. Dadurch wird eine gute Anreicherung der Aktivität bei relativ geringen Verlusten (<50 %) erreicht. Weitere Chromatographieschritte, die zweckmäßig sind, sind beispielsweise eine Heparinsepharose-Chromatographie (Miao et al., J. Neurosci. 17 (1997) 5891 - 5899), die jedoch vorzugsweise in Gegenwart von nichtionischen Detergentien durchgeführt wird. Weiter bevorzugt ist es, nach der Heparinsepharose-Chromatographie eine Dialyse, vorzugsweise gegen niedere Ionenstärken (z. B. Puffer mit 1 - 10 mmol/l Natriumphosphat, pH 6,5 - 7,5), durchzuführen. Besonders vorteilhaft ist es hierbei, daß bei dieser Dialyse der Puffer, gegen den dialysiert wird, 10 - 100 mmol/l, vorzugsweise 50 mmol/l Natriumchlorid enthält und die Dialyse bei einer geringen Konzentration des hh-Proteins (1 mg/ml oder geringer, vorzugsweise 0,5 mg/ml oder geringer) durchgeführt wird.

Es ist weiter bevorzugt, bei der Reinigung oder zumindest vor Bestimmung der Aktivität des Proteins Suramin zuzusetzen. Dadurch wird ebenfalls eine Stabilisierung der Aktivität erreicht. Für Suramin war bisher lediglich bekannt, daß es geeignet ist, hh-Proteine von der Zelloberflächen- oder der extrazellulären Matrix abzulösen (Bumcrot et al., siehe oben).

Zur weiteren Reinigung ist es bevorzugt, nochmals an Heparin-Sepharose und Hydroxylapatit zu chromatographieren.

In einer weiteren Ausführungsform der Erfindung kann die erfindungsgemäße hh-Mutante zur Herstellung einer pharmazeutischen Zusammensetzung, die ebenfalls Gegenstand der Erfindung ist, verwendet werden. Diese pharmazeutische Zusammensetzung enthält eine pharmakologisch effektive Dosis des erfindungsgemäßen Proteins und kann sowohl systemisch als auch lokal appliziert werden. Es ist ebenfalls bevorzugt, die erfindungsgemäßen Proteine in Kombination mit anderen Proteinen der Hedgehog-Familie oder Knochenwachstumsfaktoren, wie Bone Morphogenic Proteinen (BMPs), (Wozney et al., Cell. Mol. Biol. of Bone, Bone Morphogenetic Proteins and their Gene Expression, 131 - 167, Academic Press Inc. 1993) oder Parathyroid Hormonen (Karablis et al., Genes and Development 8 (1994) 277 - 289).

Das erfindungsgemäße Protein kann vorteilhaft zur Induktion von Chondrozyten und Osteozyten in einer osteoinduktiven pharmazeutischen Zusammensetzung verwendet werden. Solche osteoinduktiven pharmazeutischen Zusammensetzungen sind beispielsweise aus US-Patent 5,364,839, WO 97/35607, WO 95/16035 bekannt.

Bei einer lokalen Applikation des erfindungsgemäßen Proteins ist bevorzugt, dieses in Kombination mit einer geeigneten Matrix als Träger und/oder einem Sequestierungsagens zu verwenden. Eine solche Matrix ist dazu geeignet, das Protein, insbesondere in der Umgebung von Knochen, in vivo langsam in aktiver Form freizusetzen. Das Sequestierungsagens ist eine Substanz, welche die Applikation, beispielsweise durch Injektion, erleichtert und/oder die Migration des erfindungsgemäßen Proteins von der Applikationsstelle verhindert oder zumindest verzögert.

Als Matrixmaterial ist insbesondere ein biokompatibles degradierbares Material, beispielsweise auf Kollagenbasis oder anderen Polymeren, basierend auf Polymilchsäure, Polyglykolsäure oder Co-Polymeren aus Milchsäure und Glykolsäure geeignet. Solche Polymermatrices sind beispielsweise in der WO 93/00050 beschrieben.

Sequestierungsagentien sind beispielsweise Cellulose und celluloseartige Materialien sowie beispielsweise Alkylcellulose, Carboxymethylcellulose, Hyaluronsäure, Natriumalginat, Polyäthylenglykol und Polyvenylalkohol, wobei Hyaluronsäure, insbesondere in einer pharmazeutischen Zusammensetzung auch ohne Trägermatrix, besonders bevorzugt ist.

Zur Herstellung der pharmazeutischen Zusammensetzung ist es weiter bevorzugt, Hilfsstoffe, wie Mannitol, Sucrose, Laktose, Glucose oder Glycin sowie Antioxidantien, wie EDTA, Citrat und Detergentien, vorzugsweise nicht-ionische Detergentien, wie Polysorbate oder Polyoxyethylene, zuzusetzen.

In einer weiteren bevorzugten Ausführungsform ist eine pharmazeutische Zusammensetzung des erfindungsgemäßen Hedgehog-Proteins mit Suramin bevorzugt und diese vorteilhaft verwendbar.

Die folgenden Beispiele, Publikationen und Abbildungen erläutern die Erfindung, deren Schutzumfang sich aus den Patentansprüchen ergibt, weiter. Die beschriebenen Verfahren sind als Beispiele zu verstehen, die auch noch nach Modifikationen den Gegenstand der Erfindung beschreiben.

<u>Beschreibung der Figuren:</u>

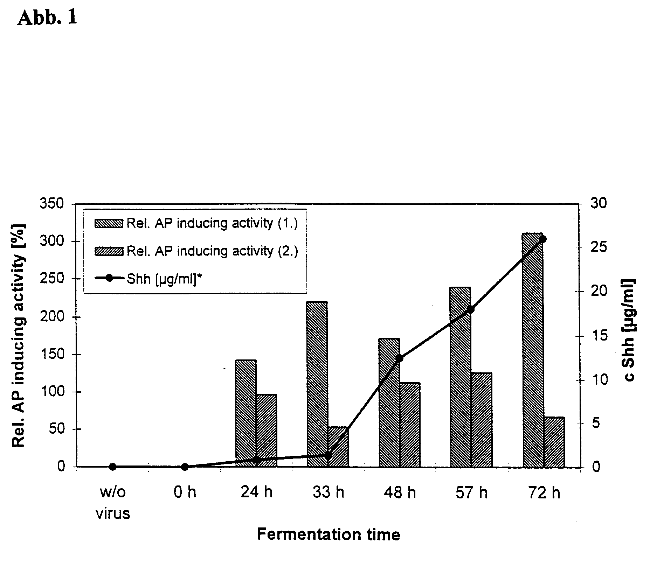

Fig. 1: Kinetik der Sekretion von alkalischer Phosphatase induzierender Aktivität (Balken) und hh-Mutante (Punkte und Linie) durch High Five Zellen nach Infektion mit Baculovirus

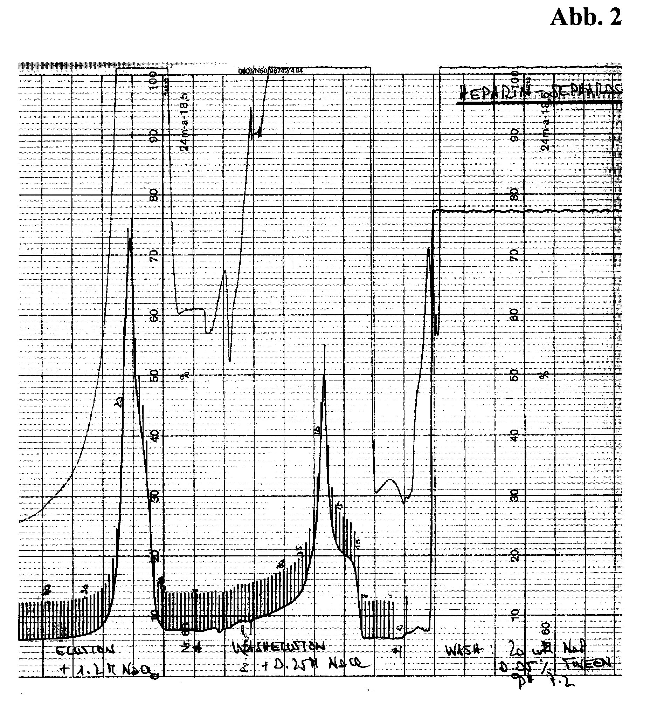

Fig. 2: Elutionsdiagramm der Reinigung des Fermentationsüberstandes mit Heparinsepharose

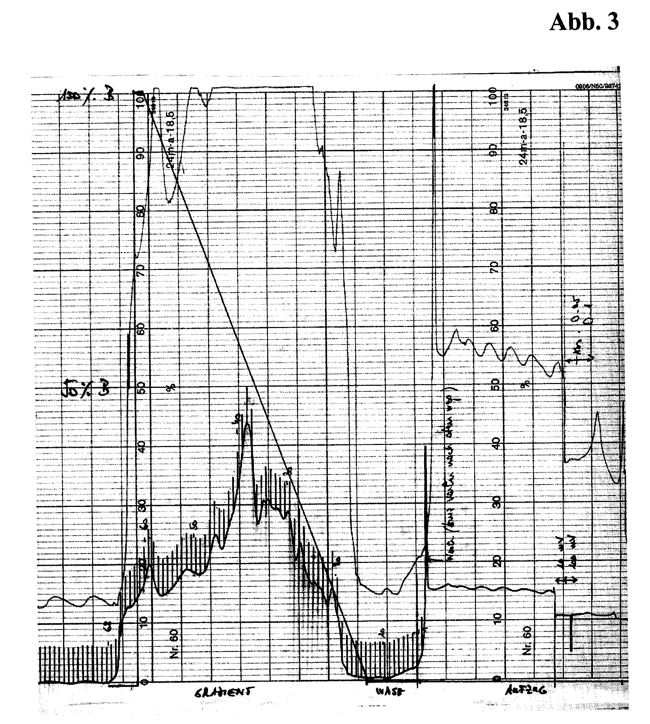

Fig. 3: Elutionsdiagramm der Reinigung des dialysierten Eluates der Heparin-Sepharose mit Hydroxylapatit

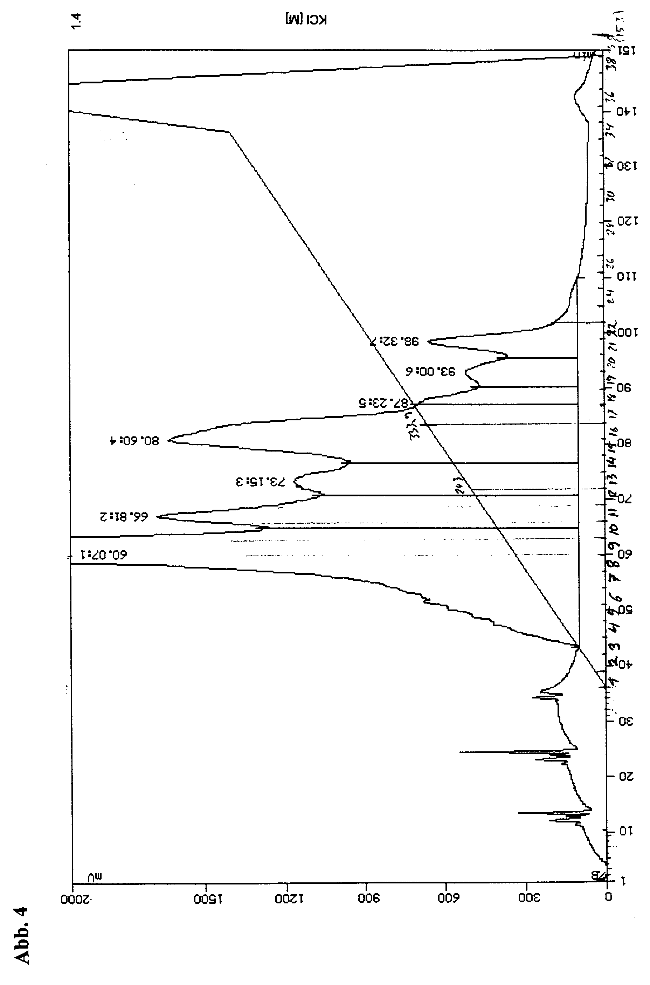

Fig. 4: Elutionsdiagramm der Reinigung der dialysierten aktiven Fraktionen der Hydroxylapatit-Säule mit einer 1 ml HiTrap Heparin-Säule

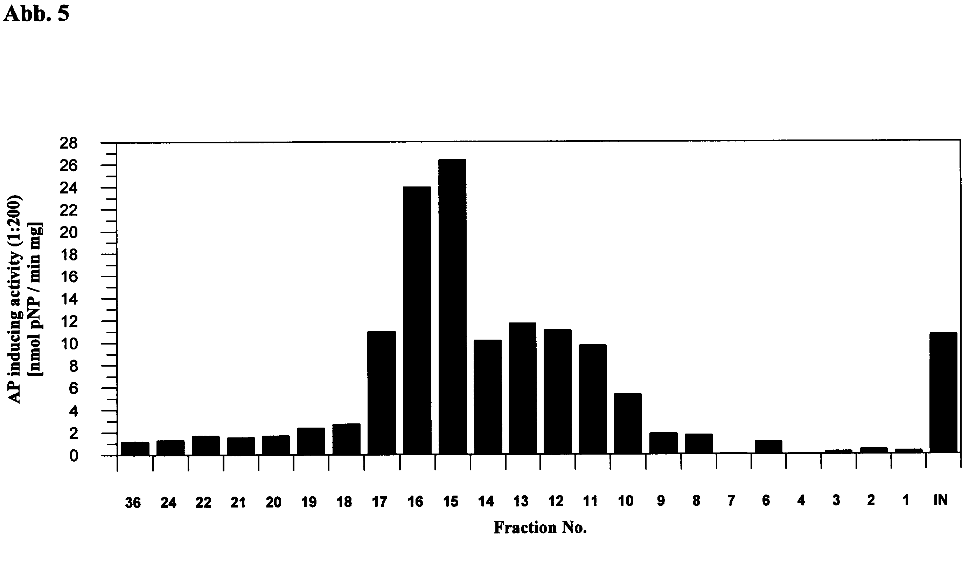

Fig. 5: Alkalische Phosphatase induzierende Aktivität der Fraktionen 1 ml High Trap Heparin Chromatographie

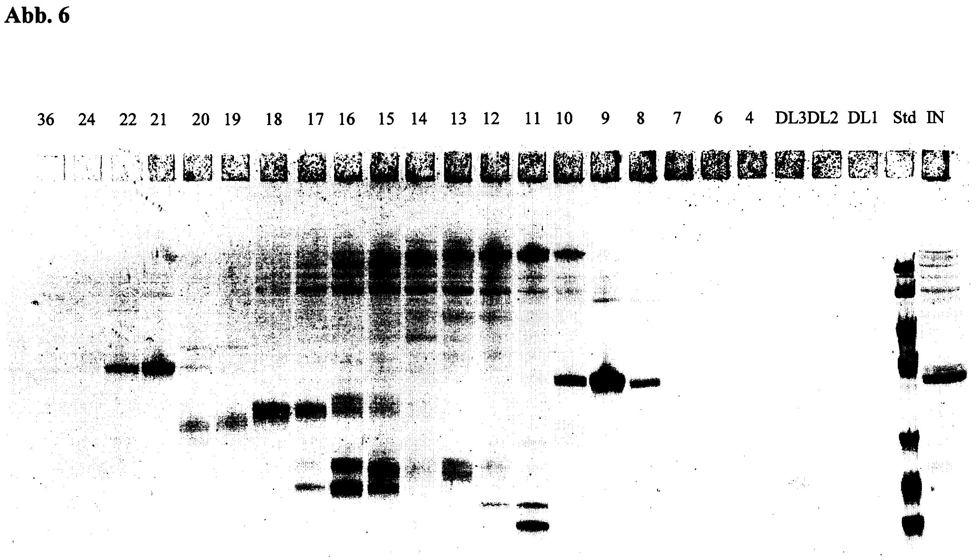

Fig. 6: Coomassie-Färbung der SDS-PAGE mit alkylierten Fraktionen der 1 ml High Trap Heparin Chromatographie

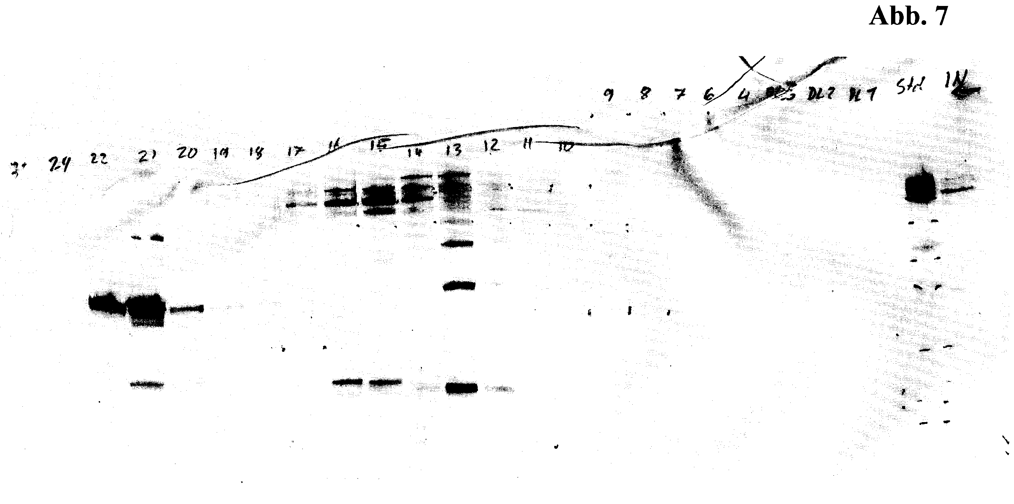

Fig. 7: Western blot mit einem Antikörper gegen den N-Terminus von shh der SDS-PAGE mit alkylierten Proben der Fraktionen der 1 ml High Trap Heparin Chromatographie

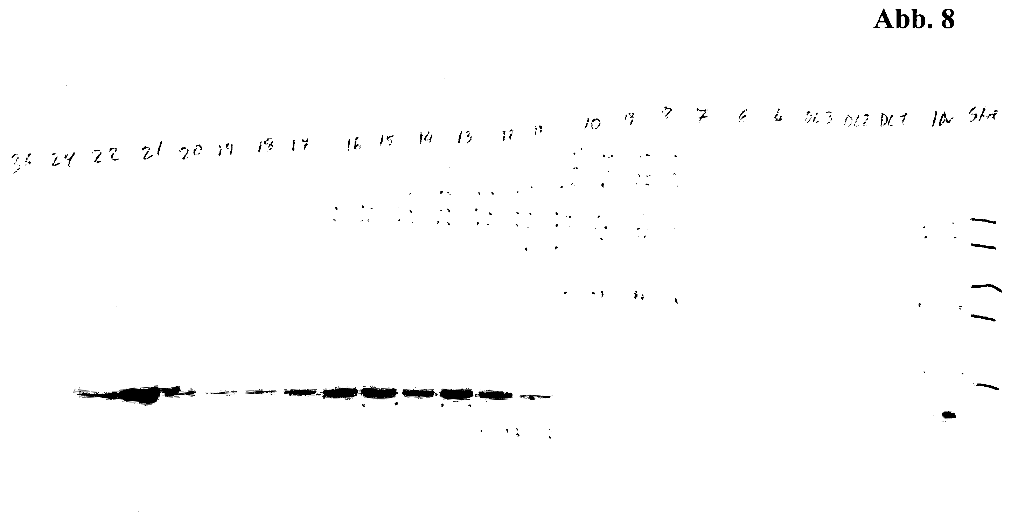

Fig. 8: Western blot mit einem Antikörper gegen den N-Terminus von shh der SDS-PAGE mit reduzierten Proben der Fraktionen der 1 ml High Trap Heparin Chromatographie

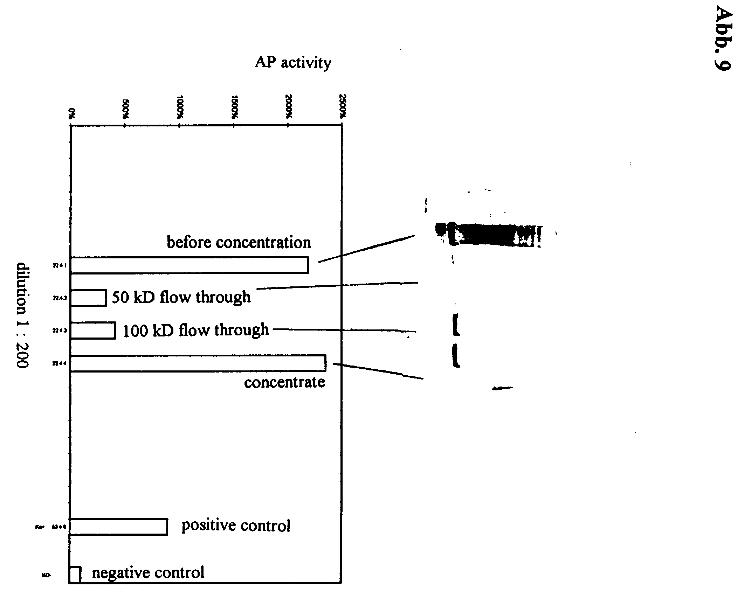

Fig. 9: Aktivität von Retentat und Permeat nach Filtration von aktiven shh Fraktionen mit Membranen mit einer Ausschlußgrenze von 30 und 100 kDa.

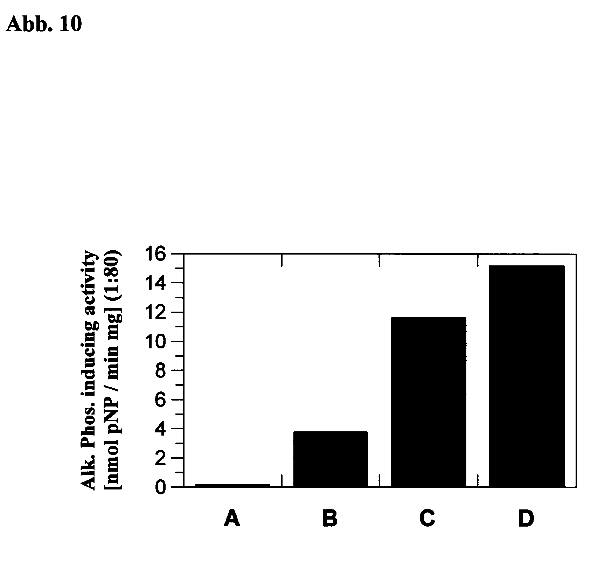

Fig. 10: Einfluß von Suramin auf die Aktivität der hh-Mutante: Zu Aliquots einer aktiver Fraktionen nach Hydroxylapatit-Chromatographie wurde kein Suramin (B), Suramin ad 0,1 mg/ml erst nach der Dialyse gegen PBS+0,05 % Tween®-80 zugegeben (C) oder Suramin ad 0,1 mg/ml vor der Dialyse zugegeben und gegen PBS+0,05 % Tween®-80 mit zusätzlich 0,1 mg/ml Suramin dialysiert.

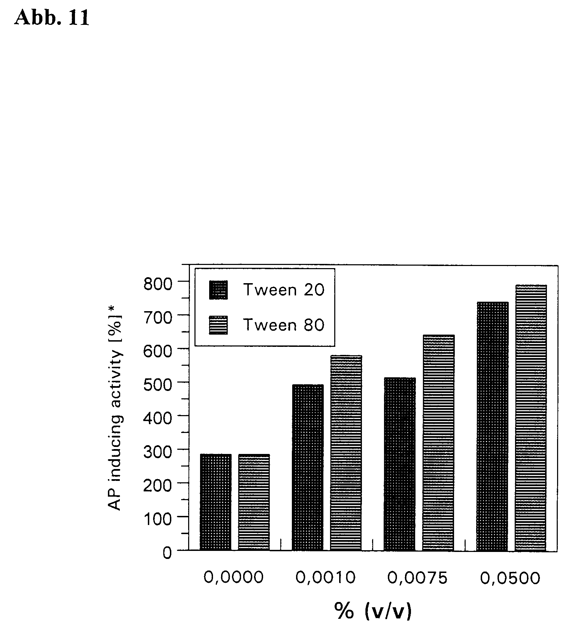

Fig. 11: Einfluß von Tween-20 und Tween-80® auf die Aktivität der hh-Mutante: Aliquots eines Pools AP aktiver Fraktionen nach SP-Sepharose-Chromatographie in 50 mM NaPi, 0,9 M NaCl, 1 mM EDTA pH 7,3 wurden zu den angegebenen Konzentrationen mit Tween versetzt und gegen PBS mit der jeweiligen Konzentration an Tween dialysiert. Vor dem Einsatz in den C3H10T1/2-Test wurden die Proben durch 0,2 μm Filter sterilfiltriert.

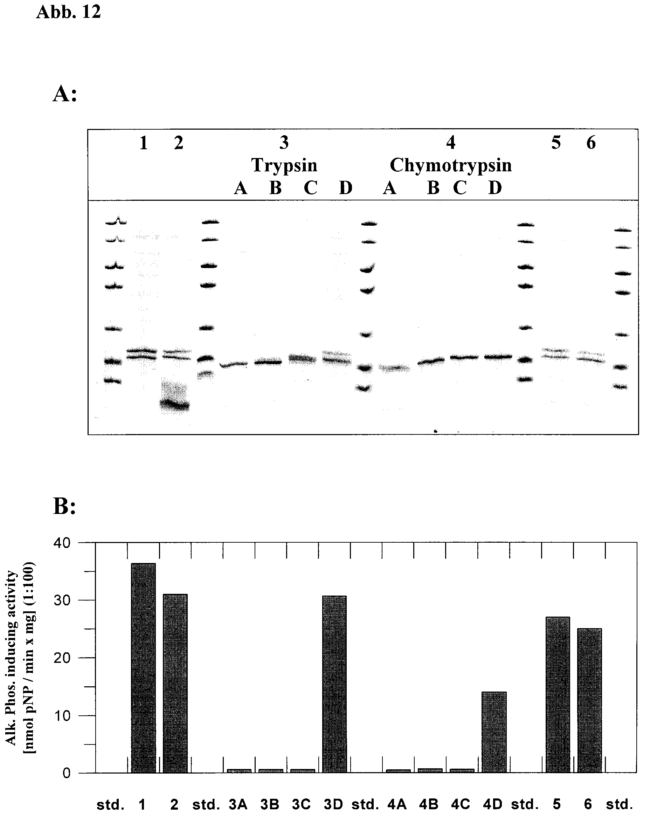

Fig. 12: Einfluß von Trypsin und Chymotrypsin auf die Aktivität der hh-Mutante: AP aktive Fraktionen nach Stufenelution von Heparin-Sepharose wurden in 10 mM NaPhosphat, 0,05% Tween®80 auf eine Proteinkonzentration von 0,46 mg/ml eingestellt und im Protease/Protein-Verhältnis (w/w) von 1:100 (A), 1:500 (B), 1:2500 (C) und 1:10000 (D) mit Trypsin , bzw. Chymotrypsin versetzt. Die Proben wurden 11 h bei RT inkubiert. Der Verdau wurde durch Zugabe von Aprotinin im 5-fachen Gewichtsüberschuß gestoppt und die Proben in der SDS-PAGE (A:) und im C3H10T1/2 -Test (B:) analysiert. 1, Einsatz; 2, Kontrolle ohne Protease; 3, Trypsin behandelte Proben; 4, Chymotrypsin behandelte Proben; 5, Kontrolle Trypsin (1:100) und Aprotinin bei t = 0; 6, Kontrolle Chymotrypsin (1:100) und Aprotinin bei t = 0.

**Beispiel 1**

**Expression von rekombinanten humanen sonic hh (shh)**

Die N-terminale Domäne von humanem shh mit den Aminosäuren 24-197 (EMBL-Accession No. L 38518) wurde wie für das Rattenprotein von Miao (J. Neurosci. (9197) 17, 5891-5899) und Bumcrot et al. (Mol.Cell.Biol. (1995) 15, 2294-2303) beschrieben mittels rekombinanten Baculovirus in High Five Zellen (Invitrogen, Leek, NL, Best.Nr. E 855-02) unter Verwendung von Excell 400 Medium (JHR, Inc.), wobei so viel Virus eingesetzt wurde, daß jede Zelle im Durchschnitt mit einem Virus infiziert wurde (multiplicity of infection (m.o.i.): 1).

Der Fermenterinhalt wurde nach 26 bzw. 72 h durch Zentrifugation mit 1000 g und Filtration geklärt und der Überstand bzw. das Permeat bis zur weiteren Verwendung bei -80°C gelagert. Proben der Fermentation wurden bzgl. ihrem Gehalt an Alkalische Phosphatase induzierender Aktivität [Nakamura et al.(1997), Kinto et al. (1997) FEBS Lett. 404, 319-323] und auf ihren Gehalt an shh Protein mittels RP-HPLC (Vydac C18, Gradient von 0-90% Acetonitril in 0,1% Trifluoressigsäure, TFA) oder SDS-PAGE analysiert.

Nach 24-32 h (bevorzugt nach 24 - 27 h) Fermentationszeit wurde die Fermentation beendet und der Überstand geklärt.

**Beispiel 2**

**Reinigung der aktiven hh-Mutante**

Der geklärte Überstand wurde nach dem Auftauen mit 1 Tablette „Complete" Inhibitor-Mix (Boehringer Mannheim, GmbH, Best.Nr. 1873580) je 50 ml Überstand versetzt und 3,5 l dieser Lösung bei 4°C auf eine Heparin-Sepharose-Säule (Volumen 90 ml; Pharmacia Biotech) aufgetragen, die zuvor mit 20 mM Natriumphosphat, pH 7,2 äquilibriert worden war. Nach dem Probenauftrag wurde mit 20 mM Natriumphosphat, 0,05 % Tween®-80, pH 7,2 (=Puffer A)gespült und unspezifisch gebundenes Protein durch einen Waschschritt mit Puffer A, der zusätzlich o,25 M NaCl enthielt, eluiert. Die Aktivität erfolgte durch eine anschließende Elution mit Puffer A, der zusätzlich 1,2M NaCl enthielt.

Dieses Eluat wurde anschließend mit einem Volumen 10 mM Natriumphosphat, 0,05 % Tween-80®, 50 mM NaCl, pH 7,2 (=Puffer B) verdünnt und gegen Puffer B bei 4oC dialysiert.

Das Dialysat wurde auf eine mit Puffer B äquilibrierte Hydroxyapatit-Säule (Volumen 10 ml; Makro Prep; 40 um, Typ I; BIO-Rad) aufgetragen. Die Elution erfolgte mit einem Gradienten von 10 bis 300 mM NaP in Puffer B (2 x 200 ml).

Aliquots der Fraktionen wurden bzgl. der Fähigkeit zur Stimulation von alkalischer Phosphatase in einer Maus-Fibroblasten Zellinie, z. B. C3H10T1/2 Zellen sowie mitells SDS-PAGE und RP-HPLC analysiert. Der Rest der Fraktionen wurde bis zur weiteren Bearbeitung bei - 80°C gelagert. Maximale Aktivität eluiert am Ende des Gradienten zwischen 0,25-0,3 M Natriumphosphat, während inaktive oder nur schwach aktive Formen von shh bereits schon wesentlich früher von der Säule eluieren.

Die aktiven Fraktionen wurden vereinigt und gegen Puffer B bei -4°C dialysiert und auf eine 1 ml HiTrap Heparin-Säule (Pharmacia Biotech) aufgetragen, die mit 20 mmol Kaliumphosphat, 0,05 Tween®80, pH 7,2 äquilibriert worden war. Die Elution erfolgte durch einen Gradienten von 0 - 1400 mM Kcl in 20 mM Kaliumphosphat, 0,05 % Tween®-80, 50 mM NaCl, pH 7,2. Aktive Fraktionen wurden durch die Stimulation von alkalischer Phosphatase in C3H10T1/2 Zellen identifiziert und alkylierte und reduzierte Proben mittels SDS-PAGE und Western blot, mit einem Antikörper gegen den N-Terminus von shh, analysiert.

Im Gegensatz zu inaktiven Fraktionen, enthalten die aktiven Fraktionen eine hh-Mutante, die unter alkylierenden Bedingungen ein um 1-3 kDa reduziertes Molekulargewicht im Vergleich zu nur schwach aktiven shh mit intakten N-Terminus zeigen. Unter reduzierenden Bedingungen hingegen ist ein derartiger Molekulargewichtsunterschled nicht nachweisbar.

**Beispiel 3**

**Induktion von alkalischer Phosphatase im Zelltest (Bestimmung der Aktivität der Alkalischen Phosphatase)**

5000 Zellen der murinen mesenchymalen pluripotenten Linie C3H10T1/2 (ATCC CCL-226) wurden pro Vertiefung einer 96-Loch Mikrotiterplatte eingesät. Die Zellen befanden sich in 100 μl DMEM, 2mM Glutamin, 100 IU/ml Penicillin, 100 ug/ml Streptomycin und 10 % fötalem Kälberserum, FKS. Am nächsten Tag wurden die zu untersuchenden Wirkstoffe in den entsprechenden Konzentrationen in einem Volumen von 100 μl zugefügt. Nach 5 Tagen wurde der Test gestoppt. Dazu wurden die Überstände abgekippt und die Zeilen einmal mit PBS gewaschen. Die Zellen wurden lysiert in 50 μl 0.1 % Triton® X-100 und bei -20 C eingefroren. Nach dem Auftauen wurden 25 μl für die Proteinbestimmung und 25 μl für die Bestimmung der Aktivität der Alkalischen Phosphatase eingesetzt.

*Proteinbestimmung nach der Anleitung des Herstellers Pierce :*

Der Ansatz wurde mit 75 μl H2O bidest. versetzt, dann wurden 100 μl BCA Protein Reagenz zugefügt (Pierce Micro BCA, Nr. 23225). Nach 60 min wurde die Optische Dichte (O.D.) bei 550 nm gemessen.

*Aktivität der Alkalischen Phosphatase nach der Anleitung des Herstellers Sigma :*

Der Ansatz wurde mit 100 μl Reaktionspuffer (Sigma 221) versetzt. Eine Substratkapsel (Sigma 104-40) wurde in 10 ml H$_2$O bidest. aufgelöst, dann wurden 100 μl zu dem Testansatz pipettiert. Die O.D. wurde nach der Gelbfärbung bei 405 nm gemessen. Bei der Reaktion setzt die Alkalische Phosphatase p-Nitrophenyl-phosphat zu p-Nitrophenol um.

Die O.D. wurden jeweils mittels Standardkurven in nmol bzw. μg umgerechnet. Die Auswertung erfolgte nach der Formel :

*nmol PNP pro (Meß)Minute pro mg (Zell)Protein*

*Ergebnisse:*

- Shh (Monomer) aus E. coli in einer Konzentration von 43 μg/ml (= 2,15 μmol/l) im Zelltest gibt 8,537 nmol pNP/min/mg Shh (Dimer) aus E. coli in einer Konzentration von 41,5 μg/ml (= 1,037 μmol/l)im Zelltest gibt 5,133 nmol pNP/min/mg
- erfindungsgemäße hh-Mutante in einer Konzentration von 0,1 μg/ml (= 5 nmol/l) mit 0,1 mg/ml Suramin im Zelltest gibt 88,762 nmol pNP/min/mg
- erfindungsgemäße hh-Mutante in einer Konzentration von 0,1 μg/ml (= 5 nmol/l) ohne Suramin im Zelltest gibt 44,828 nmol pNP/min/mg
- Kontrolle ohne Shh gibt 1,292 nmol pNP/min/mg
- in 1/40 Verdünnung eingesetzter BVCM gibt 41,961 nmol pNP/min/mg (= interne Positivkontrolle)

## Referenzliste

Asahina, J., Exp. Cell. Res. 222 (1996) 38 - 47
Bitgood, M.J. et al., Curr. Biol. 6 (1996) 296
Bumcrot et al., Mol. Cell. Biol. (1995) 2294 - 2303
Cha et al., Proc. Natl. Acad. Sci. USA 94 (1997) 10577 - 10582
Chiang, C. et al., Nature 83 (1996) 407
Fietz, M. et al., Development (Suppl.)(1994) 43 - 51
Hynes, M. et al., Neuron 15 (1995) 35 - 44
Karablis et al., Genes and Development 8 (1994) 277 - 289
Kinto et al., FEBS Letters, 404 (1997) 319 - 323
Lai, C.J. et al., Development 121 (1995) 2349.
Miao et al., J. Neurosci. 17 (1997) 5891 - 5899
Nakamura, T. et al., Biochem. Biophys. Res. Comm. 237 (1997) 465 - 469
Perrimon, N., Cell 80 (1995) 517 - 520
Porter, J.A. et al., Science 274 (1996) 255 - 259
Smith, J.C., Cell 76 (1994) 193 - 196
US-Patent 5,364,839
Vortkamp, A. et al., Science 273 (1996) 613
WO 97/35607
WO 93/00050
WO 95/16035
Wozney et al., Cell. Mol. Biol. of Bone, Bone Morphogenetic Proteins and their Gene Expression, 131 - 167 ,Academic Press Inc. 1993

**Patentansprüche**

1.  Posttranslational prozessierte Hedgehog-Protein-Mutante, welche erhältlich ist durch Expression eines Gens, welches ein Hedgehog-Protein codiert, in einem Bacculo-Virus-Expressionssystem, bei einer Fermentation über einen Zeitraum von bis zu 30 Stunden, Reinigung des Zellüberstands, in Gegenwart eines Proteaseninhibitors und eines nicht-ionischen Detergenz und Isolierung der hh-Mutante, welche an Heparinsepharose und Hydroxylapatit bindet und dadurch gekennzeichnet ist, daß diese hh-Mutante

    —  unter alkylierenden Bedingungen ein Molekulargewicht von 22 ± 1 kDa zeigt,
    —  unter reduzierenden Bedingungen ein Molekulargewicht von 24 ± 1 kD zeigt,
    —  in ihrer Aktivität durch Suramin stabilisiert wird,
    —  bei einer N-terminalen Abspaltung von 8 oder mehr Aminosäuren inaktiviert wird,
    —  bei einer Inkubation mit 10 mmol/l DTE über 2,5 Stunden bei 37°C zu 90 % oder mehr inaktiviert wird,
    —  in einer Konzentration von 5 nmol/l, in Gegenwart von Suramin, eine Aktivität für alkalische Phosphatase von ca. 90 nmol pNP/min/mg induziert und

— nicht cholesterinmodifiziert ist.

2. Verfahren zur Herstellung einer posttranslational prozessierten Hedgehog-Protein-Mutante durch Expression eines Gens, welches ein Hedgehog-Protein codiert, in einem Bacculo-Virus-Expressionssystem, bei einer Fermentation über einen Zeitraum von 24 - 27 Stunden, Reinigung des Zellüberstands, in Gegenwart eines Proteaseninhibitors und eines nicht-ionischen Detergenz und Isolierung der hh-Mutante, welche an Heparinsepharose und Hydroxylapatit bindet und dadurch gekennzeichnet ist, daß diese hh-Mutante

— unter alkylierenden Bedingungen ein Molekulargewicht von 22 ± 1 kDa zeigt,
— unter reduzierenden Bedingungen ein Molekulargewicht von 24 ± 1 kD zeigt,
— in ihrer Aktivität durch Suramin stabilisiert wird,
— bei einer N-terminalen Abspaltung von 8 oder mehr Aminosäuren inaktiviert wird,
— bei einer Inkubation mit 10 mmol/l DTE über 2,5 Stunden bei 37°C zu 90 % oder mehr inaktiviert wird,
— in einer Konzentration von 5 nmol/l, in Gegenwart von Suramin, eine Aktivität für alkalische Phosphatase von ca. 90 nmol pNP/min/mg induziert und
— nicht cholesterinmodifiziert ist.

3. Verfahren nach Anspruch 2, dadurch gekennzeichnet, daß nach der Chromatographie an Heparinsepharose eine Dialyse gegen niedrige Ionenstärken durchgeführt wird.

4. Verfahren nach Anspruch 3, dadurch gekennzeichnet, daß die Dialyse in Gegenwart von 10 - 100 mmol/l Natriumchlorid durchgeführt wird.

5. Pharmazeutische Zusammensetzung, enthaltend eine hh-Mutante, nach Anspruch 1.

6. Pharmazeutische Zusammensetzung nah Anspruch 5, enthaltend Suramin, eine biokompatible Matrix und/oder eine Sequestierungsagens.

7. Verfahren zur Herstellung einer pharmazeutischen Zusammensetzung durch Kombination einer hh-Mutante gemäß Anspruch 1, mit einem pharmazeutischen Hilfsstoff oder mit Suramin.

8. Verfahren zur Herstellung einer pharmazeutischen Zusammensetzung durch Kombination einer hh-Mutante nach Anspruch 1 mit einer biocompatiblen Matrix und/oder einem Sequestierungsagens.

**Abb. 1**

**Abb. 2**

**Abb. 3**

Abb. 4

EP 0 919 618 A1

**Abb. 5**

**Abb. 6**

Abb. 7

Abb. 8

EP 0 919 618 A1

**Abb. 9**

AP activity

before concentration

50 kD flow through

100 kD flow through

concentrate

positive control

negative control

dilution 1 : 200

Abb. 10

**Abb. 11**

Abb. 12

A:

B:

**Europäisches Patentamt**

# EUROPÄISCHER RECHERCHENBERICHT

Nummer der Anmeldung

EP 97 12 0891

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.6) |
|---|---|---|---|
| D,A | BUMCROT DA ET AL: "Proteolytic processing yields two secreted forms of sonic hedgehog 'published erratum appears in Mol Cell Biol 1995 May;15(5):2904!" MOL CELL BIOL, APR 1995, 15 (4) P2294-303, UNITED STATES, XP002062954 * das ganze Dokument * | 1-8 | C12N15/12 C12N15/86 C07K14/47 C12N5/10 A61K38/17 |
| A | WO 96 16668 A (UNIV JOHNS HOPKINS MED ;UNIV WASHINGTON (US)) * Seite 24, Zeile 25 - Seite 25, Zeile 7; Ansprüche 1-31; Abbildung 12; Beispiele 1,6,9 * * Seite 22, Zeile 27 - Seite 23, Zeile 4 * * Seite 12, Absatz 2 - Seite 13, Absatz 3 * | 1-8 | |

RECHERCHIERTE SACHGEBIETE (Int.Cl.6)

C07K
C12N

Der vorliegende Recherchenbericht wurde für alle Patentansprüche erstellt

| Recherchenort | Abschlußdatum der Recherche | Prüfer |
|---|---|---|
| DEN HAAG | 22.April 1998 | Gurdjian, D |

EPO FORM 1503 03.82 (P04C03)